(19) Europäisches Patentamt
European Patent Office
Office européen des brevets

(11) Publication number: **0 098 689**
**B1**

(12) # EUROPEAN PATENT SPECIFICATION

(45) Date of publication of patent specification: **17.12.86**

(51) Int. Cl.⁴: **C 07 C 51/54,** C 07 C 51/56, C 07 C 53/12

(21) Application number: **83303208.9**

(22) Date of filing: **03.06.83**

(54) **Process for the production of monocarboxylic acid anhydrides.**

(30) Priority: **05.06.82 GB 8216426**
**18.03.83 GB 8307610**

(43) Date of publication of application:
**18.01.84 Bulletin 84/03**

(45) Publication of the grant of the patent:
**17.12.86 Bulletin 86/51**

(84) Designated Contracting States:
**BE DE FR GB IT NL**

(56) References cited:
**EP-A-0 008 396**
**EP-A-0 011 042**
**EP-A-0 048 210**
**EP-A-0 055 622**
**US-A-2 730 546**
**US-A-4 115 444**
**US-A-4 218 340**

(73) Proprietor: **BP Chemicals Limited**
**Belgrave House 76 Buckingham Palace Road**
**London, SW1W 0SU (GB)**

(72) Inventor: **Cook, John**
**BP Chemicals Limited Hull Works Saltend**
**Hedon Hull HU12 8DS (GB)**
Inventor: **Drury, David James**
**BP Chemicals Limited Hull Works Saltend**
**Hedon Hull HU12 8DS (GB)**

(74) Representative: **Crack, Richard David et al**
**c/o The British Petroleum Company plc Patents**
**Division Chertsey Road**
**Sunbury-on-Thames Middlesex, TW16 7LN (GB)**

EP 0 098 689 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the production of monocarboxylic acid anhydrides, especially lower alkanoic acid anhydrides such as acetic anhydride.

Acetic anhydride has been known as an industrial chemical for many years. It constitutes the second largest end use for acetic acid and is extensively employed in the production of cellulose acetate and other esters. Smaller quantities are used in the production of aspirin, pharmaceuticals, pesticides and low temperature bleach activators. Uses have been proposed for other carboxylic acid anhydrides, such as propionic anhydride.

On an industrial scale acetic anhydride is currently produced either by reaction of ketene and acetic acid, the ketene being obtained either by dehydration of acetic acid or by decomposition of acetone, or by the oxidation of acetaldehyde, which also yields acetic acid. Each of these conventional routes has well-known disadvantages which knowledge, together with the increasing attention being paid in chemical syntheses involving carbon monoxide and carbon monoxide/hydrogen mixtures, has led to investigations into the production of acetic anhydride utilising these materials.

US Patent No. 2730546 describes a process for the production of aliphatic oxygen compounds such as carboxylic acid anhydrides by reacting carbon monoxide with esters of lower saturated fatty acids with saturated lower aliphatic alcohols at superatmospheric temperatures and under superatmospheric pressure in the presence as catalyst of a complex compound of a cobalt halide wherein the halogen is selected from the group consisting of bromine and iodine with an organic halide selected from the group consisting of quaternary ammonium and phosphonium bromides, iodides and bromideiodides. Suitable catalysts are said to conform to the general formula:

$$(A_4N)_2 \cdot Co \cdot X_4$$

wherein A stands for a lower molecular alkyl radical, in particular a hydrocarbon radical containing between 1 and 4 carbon atoms, or two of the A form an alkylene radical as in a pyrrolidone ring, and X stands for a halide ion, in partiuclar a bromide or iodide ion. Instead of the ammonium compounds there may also be used the corresponding phosphonium compounds. In the Examples, the molar amount of halide is of the same order as the amount of the cobalt compound, and in those Examples quoting catalyst levels, calculated from the available data catalyst efficiencies are around 1.3 mol acetic anhydride/mol Co/hour (at 200 bar/180°C). USP 2789137 describes a process for the production of acetic acid anhydride by treating in an anhydrous inert organic solvent medium (preferably N-methyl pyrrolidone), containing a catalyst consisting of a member of the group consisting of cobalt iodide, and a mixture of cobalt iodide and cobalt bromide, dimethyl ether with carbon monoxide under a pressure exceeding 400 atmospheres and at a temperature between 150 and 220°C. Disadvantages of the aforesaid processes involving the use of cobalt catalysts are the excessive pressures employed and the low catalyst efficiencies achieved.

Further, European Patent Application No. 25702 discloses a process which comprises reacting methyl acetate or dimethyl ether with hydrogen and carbon monoxide in the presence of a cobalt catalyst and an amine or phosphine and iodine to form ethylidene diacetate. Although the stated object of the process is to produce ethylidene diacetate, acetic anhydride is produced as a coproduct in some of the worked examples.

It has now been found that by the use of a particular promoter system with the cobalt and by controlling the partial pressure of hydrogen within defined limits it is possible to prepare acetic anhydride with a greater selectivity than that disclosed in the above mentioned European Patent Application.

Accordingly, the present invention provides a process for the production of a monocarboxylic acid anhydride which process comprises reacting an ether or ester of formula $R^1OR^2$ or $R^1COOR^2$ wherein $R^1$ and $R^2$, which may be the same or different, are monovalent hydrocarbyl radicals, with carbon monoxide in a reactor at elevated temperature in the presence of an effective amount of cobalt as the sole metal component and a promoter system comprising an iodide or bromide and quaternary nitrogen or phosphorus characterised in that

(1) the atomic ratio of iodide or bromide to cobalt is at least 5:1;
(2) hydrogen if present is in an amount such that its partial pressure is not more than 20% of the total pressure of carbon monoxide and hydrogen;
(3) the quaternary nitrogen or phosphorus is formed in the reactor from a first component which is a trivalent nitrogen or phosphosus and a second component which is a quaternising agent, and
(4) the reaction is carried out at a pressure in the range 0.3 to 350 bars.

By operating with the process as defined above and controlling the reaction time within appropriate limits it has been found that the product consists exclusively of acetic anhydride or that ethylidene diacetate, if coproduced, is present in the product in an amount of not more than 5% on a molar basis referred to acetic anhydride and ethylidene diacetate.

It has been found that the presence of nickel as a cocatalyst with the cobalt as described in the above European Patent Application 25702 tends to favour the production of ethylidene diacetate, thereby reducing the selectively to acetic

anhydride, and it is threefore preferred in the present invention that the catalyst should be substantially nickel-free.

Control of the reaction time can also be important, particularly when operating in the presence of hydrogen, because the latter can react with the acetic anhydride product to form ethylidene diacetate or the acetic anhydride can decompose, thereby reducing the selectivity.

With regard to the ether or carboxylate ester feed, $R^1$ and $R^2$ in the formula $R^1OR^2$ or $R^1COOR^2$ may suitably be a saturated hydrocarbyl group, e.g. an alkyl group of 1 to 11 carbon atoms, or a monocyclic aryl group, e.g. phenyl, or an alkaryl group, e.g. benzyl. Preferably $R^1$ and $R^2$ are independently lower alkyl groups of 1 to 4 carbon atoms, e.g. methyl, ethyl, n-propyl, iso-propyl, n-butyl, sec-butyl, iso-butyl and tertbutyl. Even more preferably the ester is methyl acetate or the ether is dimethyl ether and the acid anhydride produced by the process is acetic anhydride. Mixtures of ethers or carboxylate esters may be used if so desired.

The carbon monoxide may be substantially pure or it may contain other components which are either inert to the reaction components or have a beneficial effect upon the reaction. Such impurities include nitrogen, carbon dioxide, hydrogen and methane.

The cobalt may be added in any convenient form, e.g. in the zero valent state or in any higher valent form. Thus the cobalt may be added as the elemental metal in finely divided form, or as the carbonate, oxide, hydroxide, bromide, iodide, chloride, lower alkoxide such as methoxide, phenoxide, or carboxylate wherein the carboxylate anion is derived from an alkanoic acid of 1 to 20 carbon atoms. Similarly, complexes of the metal can be employed, for example the cobalt may be coordinated with one or more ligands selected from carbon monoxide, halides, phosphines, arsines and trivalent nitrogen compounds. Suitably, cobalt may be added as a halide, a carboxylate salt, a carbonyl or a carbonyl halide.

The quaternary nitrogen atom can be provided by N-methylpyridinium iodide or bromide; N,N-dimethylimidazolium iodide or bromide; N-methyl-3-picolinium iodide or bromide; N-methyl-3,4-lutidinium iodide or bromide and N-methylquinolinium iodide or bromide.

The quaternary phosphorus atom can be provided by the iodide or bromide of tetramethyl phosphonium, or ethyltrimethyl phosphonium, or methyltripropyl phosphonium, or methyltriphenyl phosphonium.

The first component can be a heterocyclic compound of trivalent nitrogen or phosphorus which is capable of forming a quaternary compound under the reaction conditions and, as a second component, a quaternising agent e.g. an alkyl iodide or an alkyl bromide.

Suitable trivalent nitrogen compounds include heterocyclic amines such as pyridine, picoline, quinoline, hydroxyquinoline, methylquinoline, pyrrole, pyrrolidine, pyrrolidone and the like, or an imidazole, such as imidazole, methylimidazole and the like. Alternatively, there may be added a compound of formula:—

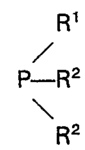

wherein $R^1$, $R^2$ and $R^3$ which may be the same or different are alkyl groups of up to 20, preferably from 1 to 8 carbon atoms or monocyclic aryl groups, or $R^3$ may be the group:

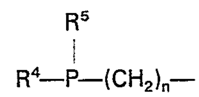

wherein $R^4$ and $R^5$ are each a monocyclic aryl group or an alkyl group and n is zero or an integer in the range 1 to 20. Suitable compounds having the above formula include trimethylphosphine, tripropylphosphine and triphenylphosphine.

Although the molar ratio of the first component to the second component in the promoter combination may vary over a wide range. It may suitably be in the range from 0.1:1 to 2:1. It has been found that the catalyst efficiency increases as the ratio approaches 1:1 from either direction. Accordingly, a molar ratio of about 0.5 to 1 to 2:1 is preferred.

In a modification the quaternary nitrogen in the form of imidazolinium can be added as such to the reaction vessel instead of being formed in situ from the first and second components.

The atomic ratio of iodide or bromide to cobalt in at least 5 and may suitably be in the range from 5:1 to 100:1, though higher ratios may be employed if desired.

A copromoter in the form of a lower monocarboxylic acid may be employed with advantage. The monocarboxylic acid suitably corresponds to the acid anhydride produced in the reaction. For example, using methyl acetate as feed to produce acetic anhydride, the monocarboxylic acid employed is preferably acetic acid.

The elevated temperature employed may suitably be in the range from 50 to 350°C, preferably from 100 to 250°C and the partial pressure of carbon monoxide may suitably be in the range from 0.3 to 350 bar, preferably form 1 to 200 bar, more preferably at least 20 bar though higher pressures may be employed if so desired under appropriate conditions.

As previously stated, the partial pressure of hydrogen is not more than 20% of the total pressure of the carbon monoxide and hydrogen, preferably not more than 10%. Although the process can be performed in the absence of hydrogen (apart from trace amounts which may be present in the carbon monoxide) in certain circumstances a small quantity of hydrogen is employed to improve the catalyst stability.

The process may be carried out in the liquid phase or in the vapour phase. In the case of both liquid and vapour phase operation, the catalyst and optionally also the promoter combination may be supported, i.e. they may be dispersed on a conventional support material, such as for example alumina, silica, silica/alumina, zeolites, clays, titania or zirconia. The catalyst and optionally the promoter may be applied to the support in conventional manner, e.g. by impregnation from solution. The catalyst concentration on the support may suitably be in the range from 0.01 to 10% by weight.

The process of the invention may be carried out batchwise or continuously.

The invention will now be further illustrated by reference to the following Examples.

In all the examples the selectivity is on a molar basis referred to acetic anhydride and ethylidene diacetate.

Example 1

Carbonylation of methyl acetate:hydrogen absent. Methyl acetate (35 g), acetic acid (15 g), methyl iodide (8 g), $Co_2(CO)_8$ (0.2 g) and 1-methylimidazole (4.6 g) were charged to a stainless steel autocalve which was pressured to 1000 psig with CO at room temperature.

The vessel was then heated and stirred for 3 hours at 200°C. At this temperature the initial pressure was approximately 1300 psig.

GC analysis of the reaction mixture showed it to contain 3.3 g of acetic anhydride and 0.02 g of ethylidene diacetate (99% selectivity).

Example 2

Carbonylation of methyl acetate:hydrogen absent. A mixture of methyl acetate (35 g), acetic acid (15 g), methyl iodide (4 g), $CO_2(CO)_8$ (0.2 g) and 1-methylimidazole (2.4 g) was charged to a stainless steel autocalve and pressured with CO(1000 psig) at room temperature.

The vessel was stirred for one hour at 150°C. At the reaction temperature the intial pressure was approximately 1150 psig.

GC analysis of the reaction mixture showed it to contain 2.3 g of acetic anhydride and 0.01 g of ethylidene diacetate (99% selectivity).

Example 3

Example 2 was repeated except 8g of methyl iodide and 4.8 g of 1-methylimidazole were used.

GC analysis of the reaction mixture showed it to contain 3.9 g of acetic anhydride and 0.05 g of ethylidene diacetate (99% selectivity).

Example 4

A glass pressure vessel was charged with a mixture of methyl acetate (25 g), methyl iodide (4 g), $CO_2(CO)_8$ (0.1 g) and 1-methylimidazole (1.1 g).

CO (100 psig) was then pressed into the vessel at room temperature. The vessel was stirred for 2 hours at 100°C.

GC analysis of the reaction mixture showed it to contain 0.1 g of acetic anhydride and no ethylidene diacetate.

Example 5

A stainless steel autoclave was charged with a mixture of methyl acetate (50 g), methyl iodide (8 g), $CO_2(CO)_8$ (0.2 g) and 2-picoline (2.4 g).

CO (850 psig) was then pressed into the autoclave at room temperature and the vessel was stirred for 4 hours at 240°C. At this temperature the initial total pressure in the reactor was approximately 1500 psig.

GC analysis of the reaction mixture showed it to contain 6.4 g of acetic anhydride and 0.01 g of ethylidene diacetate (99% selectivity).

Example 6

Carbonylation of dimethyl ether:hydrogen absent. Dimethyl ether (3.3 g) acetic acid (40 g), methyl iodide (10 g), $CO_2(CO)_8$ (0.3 g) and 1-methylimidazole (6 g) were charged to a stainless steel autoclave which was pressured to 1000 psig with CO at room temperature. The vessel was then heated and stirred for one hour at 150°C. At this temperature the initial pressure was 1100 psig.

Analysis by gas chromatography of the reaction mixture showed it to contain 0.9 g of methyl acetate and 4.2 g of acetic anhydride and 0.07 g of ethylidene diacetate (99% selectivity).

Example 7

Carbonylation of methyl acetate:hydrogen present. A stainless steel autoclave was charged with a mixture of methyl acetate (50 g), methyl iodide (8 g), $CO_2(CO)_8$ (0.2 g) and 1-methylimidazole (2.2 g).

A mixture of 200 psig $H_2$ and 850 psig CO was then pressed into the autocalve at room temperature. The vessel was stirred for 3 hours at 200°C. At this temperature the initial total pressure in the reactor was approximately 1550 psig.

Gas Chromatography analysis of the reaction mixture showed it to contain 1.1 g of acetic anhydride and 0.03 g of ethylidene diacetate (99% selectivity).

Example 8

Carbonylation of methyl acetate:hydrogen present. Methyl acetate (35 g), acetic acid (15 g), methyl iodide (8 g), $Co_2(CO)_8$ (0.2 g) and 1-methylimidazole (2.2 g) were charged to a stainless steel autoclave which was pressured to 110 psig with $H_2$ and to a further 990 psig with CO at room temperature. The vessel was then heated and stirred for 40 minutes at 115°C. At this temperature the initial total pressure was approximately 1200 psig.

GC analysis of the reaction mixture showed it to contain 1.6 g of acetic anhydride and 0.03 g of ethylidene diacetate (99% selectivity).

Example 9

Example 8 was repeated except that 4.9 g of 1-methylimidazole were used.

GC analysis of the reaction mixture showed it to contain 3.9 g of acetic anhydride and 0.06 g of ethylidene diacetate (99% selectivity).

Example 10

Carbonylation of methyl acetate:hydrogen present. A glass pressure vessel was charged with a mixture of methyl acetate (25 g), methyl iodide (4 g), $Co_2(CO)_8$ (0.1 g) and 1-methylimidazole (1.1 g).

A mixture of 10 psig $H_2$ and 90 psig CO was then pressed into the vessel at room temperature. The vessel was stirred for 2 hours at 100°C.

GC analysis of the reaction mixture showed it to contain 0.1 g of acetic anhydride and no ethylidene diacetate.

The advantage of forming the quaternary nitrogen in situ in the reaction vessel is that it avoids the necessity of having to preform the material which is not normally available in commercial quantities. Further these materials are often hygroscopic and can decompose on storage. It is also easier and more convenient to feed the two components which are normally liquids rather than the salt which in most cases is a solid. The advantage of the imidazolinium salts is that they are soluble at room temperature in the reaction medium.

**Claims**

1. A process for the production of a monocarboxylic acid anhydride which process comprises reacting an ether or ester of formula $R^1OR^2$ or $R^1COOR^2$ wherein $R^1$ and $R^2$, which may be the same or different, are monovalent hydrocarbyl radicals, with carbon monoxide in a reactor at elevated temperature in the presence of an effective amount of cobalt as the sole metal component and a promoter system comprising an iodide or bromide and quaternary nitrogen or phosphorus characterised in that

(1) the atomic ratio of iodide or bromide to cobalt is at least 5:1;
(2) hydrogen if present is in an amount such that its partial pressure is not more than 20% of the total pressure of carbon monoxide and hydrogen;
(3) the quaternary nitrogen or phosphorus is formed in the reactor from a first component which is a trivalent nitrogen or phosphorus and a second component which is a quaternising agent, and
(4) the reaction is carried out at a pressure in the range 0.3 to 350 bars.

2. A process as claimed in claim 1 characterised in that the quaternary nitrogen is imidazolium and that it is added as such to the reactor instead of being formed *in situ*.

3. A process as claimed in claim 1 characterised in that the trivalent nitrogen is a compound containing a heterocyclic ring.

4. A process as claimed in claim 3 characterised in that the trivalent nitrogen is a compound selected from pyridine, picoline, quinoline, hydroxyquinoline, methylquinoline, pyrrole, pyrrolidine, pyrrolidine or an imidazole.

5. A process as claimed in claim 1 characterised in that the reaction is carried out at a pressure in the range 1 to 200 bars.

6. A process as claimed in anyone of the preceding claims characterised in that the ether or ester is dimethyl ether or methyl acetate and the monocarboxylic acid anhydride is acetic anhydride.

7. A process as claimed in claim 1 characterised in that reaction conditions and reaction time are controlled such that the selectivity to acetic anhydride in at least 95% on a molar basis referred to acetic anhydride and ethylidene diacetate.

8. A process as claimed in claim 7 characterised in that the selectivity to acetic anhydride is at least 98% on a molar basis referred to acetic anhydride and ethylidene diacetate.

9. A process as claimed in claim 1 characterised in that the hydrogen if present is in an amount such that its partial pressure is less than 10% of the total pressure of carbon monoxide and hydrogen.

10. A process as claimed in claim 1 characterised in that the atomic ratio of iodide or bromide to cobalt is in the range 5:1 to 100:1.

**Patentansprüche**

1. Verfahren zur Herstellung eines Monocarbonsäureanhydrids, wobei das Verfahren die Umsetzung eines Ethers oder Esters der Formel $R^1OR^2$ oder $R^1COOR^2$, worin $R^1$ und $R^2$, die gleich oder unterschiedlich sein können, monovalente Kohlenwasserstoffreste sind, mit Kohlenmonoxid in einem Reaktor bei erhöhter Temperatur in Gegenwart einer wirksamen Menge von Cobalt als der einzigen Metallkomponente und eines Promotorsystems, das ein Iodid oder Bromid und quaternären Stickstoff oder Phosphor beinhaltet, umfaßt, dadurch gekennzeichnet, daß

(1) das Atomverhältnis von Iodid oder Bromid zu Cobalt mindestens 5:1 ist,
(2) Wasserstoff, falls vorhanden, in einer solchen Menge vorhanden ist, daß sein Partialdruck nicht mehr als 20% des Gesamtdruckes von Kohlenmonoxid und Wasserstoff beträgt,
(3) der quaternäre Stickstoff oder Phosphor in dem Reaktor aus einer ersten Komponente, die ein dreiwertiger Stickstoff oder Phosphor ist und einer zweiten Komponente, die ein Quaternisierungsmittel ist, gebildet wird, und
(4) die Umsetzung bei einem Druck in dem Bereich von 0,3 bis 350 bar durchgeführt wird.

2. Verfahren nach Anspruch 1, dadurch gekennzeichnet, daß der quaternäre Stickstoff Imidazolium ist und, daß es als solches zu dem Reaktor zugegeben wird, anstatt daß es *in situ* gebildet wird.

3. Verfahren nach Anspruch 1, dadurch gekennzeichnet, daß der dreiwertige Stickstoff eine Verbindung, die einen heterozyklischen Ring enthält, ist.

4. Verfahren nach Anspruch 3, dadurch gekennzeichnet, daß der dreiwertige Stickstoff eine Verbindung ist, die ausgewählt ist unter Pyridin, Pikolin, Chinolin, Hydroxychinolin, Methylchinolin, Pyrrol, Pyrrolidin, Pyrrolidon oder einem Imidazol.

5. Verfahren nach Anspruch 1, dadurch gekennzeichnet, daß die Umsetzung bei einem Druck in dem Bereich von 1 bis 200 bar durchgeführt wird.

6. Verfahren nach einem der vorhergehenden Ansprüche, dadurch gekennzeichnet, daß der Ether oder Ester Dimethylether oder Methylacetat ist und das Monocarbonsäureanhydrid Essigsäureanhydrid ist.

7. Verfahren nach Anspruch 1, dadurch gekennzeichnet, daß die Umsetzungsbedingungen und die Umsetzungszeit so geregelt werden, daß die Selektivität gegenüber Essigsäureanhydrid mindestens 95% auf einer molaren Basis bezogen auf Essigsäureanhydrid und Ethylidendiacetat beträgt.

8. Verfahren nach Anspruch 7, dadurch gekennzeichnet, daß die Selektivität gegenüber Essigsäureanhydrid mindestens 98% auf einer molaren Basis bezogen auf Essigsäureanhydrid und Ethylidendiacetat beträgt.

9. Verfahren nach Anspruch 1, dadurch gekennzeichnet, daß der Wasserstoff, falls vorhanden, in einer solchen Menge vorhanden ist, daß sein Partialdruck weniger als 10% des Gesamtdruckes des Kohlenmonoxids und Wasserstoffs beträgt.

10. Verfahren nach Anspruch 1, dadurch gekennzeichnet, daß das Atomverhältnis von Iodid oder Bromid zu Cobalt in dem Bereich von 5:1 bis 100:1 liegt.

**Revendications**

1. Procédé pour la production d'un anhydride d'acide monocarboxylique, ce procédé comprenant la reaction d'un éther ou ester de formule $R^1OR^2$ ou $R^1COOR^2$, dans lesquelles $R^1$ et $R^2$, qui peuvent être identiques ou différents, représentent des radicaux hydrocarbyles monovalents, avec le monoxyde de carbone dans un réacteur à température élevée en présence d'une quantité efficace de cobalt comme seul composant métallique et d'un système de promoteur comprenant un bromure ou iodure et de l'azote ou du phosphore quaternaire, procédé caractérisé en ce que:

(1) le rapport atomique de l'iodure ou bromure au cobalt est au moins égal à 5:1;

(2) si. l'hydrogène est présent, il l'est en une quantité telle que sa pression partielle ne représente pas plus de 20% de la pression totale du monoxyde de carbone et de l'hydrogène;

(3) le composé d'azote ou phosphore quaternaire est formé dans le réacteur à partir d'un premier composant qui est un composé d'azote ou phosphore trivalent et d'un second composant qui est un agent de quaternisation, et

(4) on effectue la réaction à une pression comprise entre 0,3 et 350 bars.

2. Procédé selon la revendication 1, caractérisé en ce que l'azote quaternaire est un imidazolium et en ce qu'il est ajouté tel quel au réacteur au lieu d'être forme "in situ".

3. Procédé selon la revendication 1, caractérisé en ce que le composé d'azote trivalent est un composé contenant un noyau hétérocyclique.

4. Procédé selon la revendication 3, caractérisé en ce que le compose d'azote trivalent est un composé choisi parmi la pyridine, la picoline, la quinoléine, une hydroxyquinoléine, une méthylquinoléine, le pyrrole, la pyrrolidine, la pyrrolidone ou un imidazole.

5. Procédé selon la revendication 1, caractérisé en ce qu'on effectue la réaction à une pression comprise entre 1 et 200 bars.

6. Procédé selon l'une quelconque des revendications précédentes, caractérisé en ce que l'éther ou l'ester est l'éther de diméthyle ou l'acétate de méthyle, et en ce que l'anhydride d'acide monocarboxylique est l'anhydride acétique.

7. Procédé selon la revendication 1, caractérisé en ce qu'on régle les conditions de réaction et le temps de réaction de manière que la sélectivité d'obtention de l'anhydride acétique soit au moins égale à 95% sur une base molaire par rapport à l'anhydride acétique et au diacétate d'éthylidène.

8. Procédé selon la revendication 7, caractérisé en ce que la sélectivité d'obtention de l'anhydride acétique est d'au moins 93% sur base molaire par rapport à l'anhydride acétique et au diacétate d'éthylidène.

9. Procédé selon la revendication 1, caractérisé en ce que, s'il est présent, l'hydrogène l'est en une quantité telle que sa pression partielle représente moins de 10% de la pression totale du monoxyde de carbone et de l'hydrogène.

10. Procédé selon la revendication 1, caractérisé en ce que le rapport atomique de l'iodure ou bromure au cobalt se situe entre 5:1 et 100:1.